# EUROPEAN PATENT APPLICATION

(11) **EP 4 406 548 A1**
(43) Date of publication of application: **31.07.2024**
(21) Application number: 22871850.8
(22) Date of filing: 13.09.2022
(51) Int. Cl.: A61K 38/22, A61P 3/10

(54) **PHARMACEUTICAL COMPOSITION CONTAINING PEGYLATED EXENATIDE VARIANT AND USE THEREOF**

(30) Priority: 24.09.2021 CN 202111126033
(71) Applicant: Pegbio Co., Ltd., Suzhou, Jiangsu 215123 (CN)
(72) Inventor: XU, Michael Min, Suzhou, Jiangsu 215123 (CN); HOU, Yinju, Suzhou, Jiangsu 215123 (CN)
(74) Representative: Schnappauf, Georg
(86) International application number: PCT/CN2022/118429
(87) International publication number: WO 2023/045802

(57) **Abstract**

The present invention relates to the technical field of protein drugs, and in particular to a pharmaceutical composition containing a pegylated exenatide variant and a use thereof. Specifically, the pharmaceutical composition provided in the present invention contains a pegylated exenatide variant and a pH regulator. In the present invention, by screening different components and concentrations, a pharmaceutical composition having excellent stability is developed; the pharmaceutical composition can be used as a drug for preventing and/or treating diabetes, and is suitable for long-term storage.

## Description

### CROSS REFERENCE TO RELATED APPLICATIONS

This application claims the priority of Chinese Patent Application No. 202111126033.7, filed with the China National Intellectual Property Administration on September 24, 2021, and titled with "PHARMACEUTICAL COMPOSITION CONTAINING PEGYLATED EXENATIDE VARIANT AND USE THEREOF", and the disclosure of which is hereby incorporated by its reference.

### FIELD

The present invention relates to the field of protein drugs, particularly to a pharmaceutical composition comprising a PEGylated exenatide variant and use thereof.

### BACKGROUND

With the development of economy and society, the extension of the average life of the population and the change of lifestyle, diabetes has become a major health care problem in all countries of the world. The incidence of diabetes is rising sharply in both developed and developing countries. Diabetes is a metabolic disease characterized by elevated blood glucose, which is caused by the deficiency in secretion function of pancreas or insulin functions. If the blood glucose of diabetic patients is not well controlled for a long time, it can lead to damage of organ and tissue, and thus it becomes the "source of all diseases", accompanied by various organic damages, especially the damage of eye, heart, blood vessel, kidney and nerves, or organ dysfunction or failure, which can cause disability or death of patients in severe cases. According to different pathogenesis, diabetes is mainly divided into type I diabetes, type II diabetes, gestational diabetes and other types of diabetes. Patients with type II diabetes are the main part of diabetic patients, accounting for about 90% of the diabetic patients. Type II diabetes is characterized by dysregulation of insulin secretion or action and β-cell dysfunction, which lead to metabolic disorder of fat, carbohydrate and protein, resulting in chronic hyperglycemia, and ultimately causing various vascular and organ complications.

Glucagon-like peptide-1 (GLP-1) was first discovered in 1985. GLP-1 is expressed by the proglucagon gene after ingestion of foods and mainly secreted by intestinal mucosa L-cells. It can stimulate islet β-cells to secrete insulin, which plays an important role in stabilizing blood glucose level (Knudsen. J. Med. Chem. 2004, 47, 17, 4128-4134). However, GLP-1 is easily degraded by dipeptidyl peptidase IV (DPPIV) *in vivo* with a half-life of less than 2 minutes, and thus almost impossible to be an effective anti-diabetic drug.

Exenatide (also called Exendin-4) is a polypeptide found in the saliva secretion of reptile venomous lizard, and has high homology (53%) with GLP-1(7-36) (Eng et al. J. Biol. Chem. 265(33), 20259-62; Eng et al. J. Biol. Chem. 267(11), 7402-5). Studies have shown that Exendin-4 can also bind to GLP-1 receptor and show similar agonistic effect as GLP-1 in pharmacology, such as increasing the synthesis of insulin and the secretion of glucose-dependent insulinotropic polypeptide; stimulating the proliferation and regeneration of β cells and inhibiting the apoptosis of β cells, thus increasing the number of β cells; inhibiting the secretion of glucagon; inhibiting the production of glycogen while avoiding severe hypoglycemia; inhibiting the postprandial gastrointestinal motility and secretion function; reducing appetite and reducing the intake of foods; and protecting nerve cells (Bonner-Weir et al. Nat Biotechnol. 23(7), 857-61; Raufman et al. J. Biol. Chem. 266(5), 2897-2902; Kolligs et al. Diabetes. 44(1), 16-19; Turton et al. Nature. 379(6560), 69-72). The effect of Exendin-4 in promoting insulin secretion and inhibiting postprandial glucagon secretion is blood glucose-dependent. Since Exendin-4 is not easy to cause hypoglycemia, it is superior to the currently used sulfonylurea hypoglycemic, which greatly reduces the times of blood glucose monitoring and reduces body weight. Exendin-4 (common name: Exenatide, trade name: Byetta), which is injected twice a day, was jointly developed by Amylin and Eli Lilly, and was marketed in the United States and Europe in 2005 and 2006 respectively, which made this kind of drugs widely used in the treatment of diabetes and obesity. Compared with natural GLP-1, Exenatide effectively reduces the enzymatic hydrolysis by DPPIV through an amino acid substitution at position 2. And the half-life of Exenatide can reach 60-90 minutes *in vivo.*

Polymer modification technology is a powerful modification technology developed in 1970s, especially represented by PEGylation technique. By this technique, polyethylene glycol (PEG) is chemically conjugated with protein drugs to modify the surface of protein. Through PEG modification, on the one hand, increasing the molecular weight of protein, and decreasing the excretion rate of protein in kidney; on the other hand, the coupled PEG chain produces steric hindrance effect on the surface of the modified protein molecule, which reduces hydrolysis effect of protein hydrolase on protein in blood, thus effectively prolonging the retention time of the protein in the circulatory system, leading to prolonged drug plasma cycle and increased systemic drug exposure and improved the therapeutic effect.

WO 2010/121559 A1 discloses a class of PEGylated exenatide variant compounds. Compared with exenatide, the conjugates obtained by specific modification with polyethylene glycol have obvious advantages in action duration, bioavailability and therapeutic effect. Clinical studies show that PEGylated exenatide variants have excellent efficacy and safety for patients with type II diabetes.

At present, polypeptide drugs represented by GLP-1 receptor agonists are mostly preserved and used in the dosage form of injection. The active ingredients, i.e. polypeptides, are easily influenced by the external environment, resulting in chemical changes such as hydrolysis, deamination, oxidation and racemization, and the impurities produced can seriously affect the effectiveness and safety of drugs. In order to solve this technical problem, in GLP-1 drugs that have been approved for marketing, an additional buffer system is usually added to the prescription to reduce the effect of external environment on the pH of the solution, so as to maintain the stability of the active ingredients. For example, the phosphate buffer system is used in Liraglutide (trade name Victoza) and Semaglutide (trade name Ozempic) (see drug instructions of Victoza and Ozempic), and the acetate buffer system is used in Exenatide (trade name Byetta) (see drug instruction of Byetta). However, the injection formulations of PEGylated exenatide variants suitable for long-term storage have not been disclosed yet.

### SUMMARY

### Problem to be solved by the present invention

In view of the fact that the injection formulations of PEGylated exenatide variant suitable for long-term storage has not been disclosed at present, the present invention provides a stable pharmaceutical composition comprising a PEGylated exenatide variant. The present invention also provides the use of the pharmaceutical composition.

### Solutions to the problem

[1]. A pharmaceutical composition comprising a PEGylated exenatide variant comprising a PEGylated exenatide variant and a pH regulator,
   wherein the structure of the PEGylated exenatide variant is: 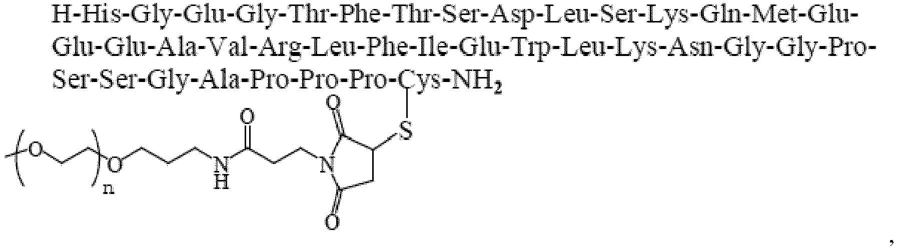 and
   the PEGylated exenatide variant comprises a polyethylene glycol modified part with 5-100kDa of molecular weight.
[2]. The pharmaceutical composition according to [1], wherein the molecular weight of the polyethylene glycol modified part is 10-50kDa.
[3]. The pharmaceutical composition according to [1] or [2], wherein the molecular weight of the polyethylene glycol modified part is 15-40kDa.
[4]. The pharmaceutical composition according to any one of [1] to [3], wherein the molecular weight of the polyethylene glycol modified part is 20-29kDa.
[5]. The pharmaceutical composition according to any one of [1] to [4], wherein the pharmaceutical composition comprises a solvent.
[6]. The pharmaceutical composition according to [5], wherein the pH of the pharmaceutical composition is 2.0-6.0.
[7]. The pharmaceutical composition according to [5] or [6], wherein the pH of the pharmaceutical composition is 2.5-5.5.
[8]. The pharmaceutical composition according to any one of [5] to [7], wherein the pH of the pharmaceutical composition is 3.0-5.0.
[9]. The pharmaceutical composition according to any one of [5] to [8], wherein the pH of the pharmaceutical composition is 3.5-4.5.
[10]. The pharmaceutical composition according to any one of [1] to [9], wherein the pH regulator is an acid.
[11]. The pharmaceutical composition according to [10], wherein the acid is selected from the group consisting of citric acid, acetic acid, trifluoroacetic acid, phosphoric acid, hydrochloric acid, tartaric acid, malic acid, propionic acid and a mixture thereof.
[12]. The pharmaceutical composition according to [10] or [11], wherein the acid is selected from the group consisting of citric acid, acetic acid, trifluoroacetic acid, phosphoric acid and a mixture thereof.
[13]. The pharmaceutical composition according to any one of [10] to [12], wherein the acid is citric acid.
[14]. The pharmaceutical composition according to any one of [1] to [9], wherein the pH regulator is a buffer system.
[15]. The pharmaceutical composition according to [14], wherein the buffer system is selected from the group consisting of glycine/hydrochloric acid, citric acid/sodium citrate, citric acid/disodium hydrogen phosphate, acetic acid/sodium acetate, lactic acid/sodium hydroxide, lactic acid/sodium carbonate, propionic acid/sodium propionate, succinic acid/sodium succinate and malic acid/sodium hydroxide.
[16]. The pharmaceutical composition according to [14] or [15], wherein the buffer system is selected from the group consisting of glycine/hydrochloric acid, acetic acid/sodium acetate, lactic acid/sodium hydroxide, lactic acid/sodium carbonate, propionic acid/sodium propionate, succinic acid/sodium succinate and malic acid/sodium hydroxide.
[17]. The pharmaceutical composition according to any one of [14] or [16], wherein the buffer system is selected from the group consisting of lactic acid/sodium hydroxide and lactic acid/sodium carbonate.
[18]. The pharmaceutical composition according to any one of [5] to [17], wherein the concentration of the PEGylated exenatide variant is 0.1-5.0 mg/ml in peptide content.
[19]. The pharmaceutical composition according to any one of [5] to [18], wherein the concentration of the PEGylated exenatide variant is 0.25-1.5 mg/ml in peptide content.
[20]. The pharmaceutical composition according to any one of [5] to [19], wherein the concentration of the PEGylated exenatide variant is 0.3-0.8 mg/ml in peptide content.
[21]. The pharmaceutical composition according to any one of [5] to [20], wherein the concentration of the PEGylated exenatide variant is 0.4-0.6 mg/ml in peptide content.
[22]. The pharmaceutical composition according to any one of [5] to [21], wherein the concentration of the PEGylated exenatide variant is 0.5 mg/ml in peptide content.
[23]. The pharmaceutical composition according to any one of [1] to [22], wherein the pharmaceutical composition comprises an osmotic pressure regulator.
[24]. The pharmaceutical composition according to [23], wherein the osmotic pressure regulator is selected from the group consisting of mannitol, sorbitol, xylitol, glycerine, sodium chloride and a mixture thereof.
[25]. The pharmaceutical composition according to [23] to [24], wherein the osmotic pressure regulator is mannitol.
[26]. The pharmaceutical composition according to any one of [23] to [25], wherein the concentration of the osmotic pressure regulator is 5-100 mg/ml.
[27]. The pharmaceutical composition according to any one of [23] to [26], wherein the concentration of the osmotic pressure regulator is 10-80 mg/ml.
[28]. The pharmaceutical composition according to any one of [23] to [27], wherein the concentration of the osmotic pressure regulator is 30-60 mg/ml.
[29]. The pharmaceutical composition according to any one of [23] to [28], wherein the concentration of the osmotic pressure regulator is 40-50 mg/ml.
[30]. The pharmaceutical composition according to any one of [1] to [29], wherein the pharmaceutical composition further comprises a bacteriostat.
[31]. The pharmaceutical composition according to [30], wherein the bacteriostat is selected from the group consisting of m-Cresol, phenol, chlorocresol, chlorobutanol, benzyl alcohol, paraben and a mixture thereof.
[32]. The pharmaceutical composition according to [30] or [31], wherein the bacteriostat is selected from the group consisting of m-Cresol, phenol, chlorocresol, chlorobutanol, paraben and a mixture thereof.
[33]. The pharmaceutical composition according to any one of [30] to [32], wherein the bacteriostat is m-Cresol.
[34]. The pharmaceutical composition according to any one of [30] to [33], wherein the concentration of the bacteriostat is 0.1-5 mg/ml.
[35]. The pharmaceutical composition according to any one of [30] to [34], wherein the concentration of the bacteriostat is 1.0-4.0 mg/ml.
[36]. The pharmaceutical composition according to any one of [30] to [35], wherein the concentration of the bacteriostat is 1.5-3.0 mg/ml.
[37]. The pharmaceutical composition according to any one of [30] to [36], wherein the concentration of the bacteriostat is 2.0-2.5 mg/ml.
[38]. The pharmaceutical composition according to any one of [1] to [37], wherein the pharmaceutical composition is an injection.
[39]. Use of the pharmaceutical composition according to any one of [1] to [38] in the manufacture of a medicament for preventing and/or treating diabetes.

### The technical effect of the present invention

In order to solve the problem of poor stability of the injection comprising PEGylated exenatide variants during long-term storage, by screening various components and concentrations, the present invention develops a pharmaceutical composition with excellent stability suitable for long-term storage.

Furthermore, under the appropriate pH condition, the pharmaceutical composition without buffer system can achieve equivalent or even better stability, which is unexpected.

### DETAILED DESCRIPTION

Various exemplary embodiments, features and aspects of the present invention will be described in detail below. The exclusive word "exemplary" here means "serving as an example, embodiment or illustration". Any embodiment described herein as "exemplary" is not necessarily to be construed as being superior or better than other embodiments.

In addition, in order to better illustrate the present invention, numerous details are given in the following particular embodiments. It should be understood by those skilled in the art that the present invention may be practiced without certain details. In other embodiments, methods, means, devices and steps well known to those skilled in the art have not been described in detail in order to highlight the gist of the present invention.

Unless otherwise specified, the units used in the specification are all international standard units, and the value and the range of value appearing in the present invention should be understood as including the inevitable systematic errors in industrial manufacture.

In this specification, the range of value indicated by "value A to value B" refers to the range including the endpoint values A and B.

In this specification, the meaning of "may/can" includes both the meaning of carrying out certain treatment and the meaning of not carrying out certain treatment.

In this specification, references to "some specific/preferred embodiments", "other specific/preferred embodiments" and "embodiments" mean that the specific elements (such as features, structures, properties and/or characteristics) described in connection with this embodiment are included in at least one embodiment described herein, and may or may not exist in other embodiments. In addition, it should be understood that described elements may be combined in any suitable manner in the various embodiments.

### First aspect of the present invention

In a first aspect, the present invention provides a pharmaceutical composition comprising a PEGylated exenatide variant and a pH regulator.

Through screening of the above-mentioned components and concentrations, the present invention develops a pharmaceutical composition with excellent stability, which is suitable for long-term preservation.

### PEGylated exenatide variant

The active ingredient of the pharmaceutical composition provided by the present invention is PEGylated exenatide variant. PEGylated exenatide variant refers to a conjugate obtained by specific modification of exenatide variant by PEGylation.

Among them, the term "exenatide variant" refers to Cys³⁹-Exendin-4 (or Cys³⁹-exenatide), which is a variant obtained by substituting serine (Ser) at the 39th position in the amino acid sequence of exenatide with cysteine (Cys). Specifically, the structure of Cys³⁹-Exendin-4 (or Cys³⁹-exenatide) is as follows: H-His-Gly-Glu-Gly-Thr-Phe-Thr-Ser-Asp-Leu-Ser-Lys-Gln-Met-Glu-Glu-Glu-Ala-Val-Arg-Leu -Phe-Ile-Glu-Trp-Leu-Lys-Asn-Gly-Gly-Pro-Ser-Ser-Gly-Ala-Pro-Pro-Pro-Cys-NH₂, as shown in SEQ ID NO.1.

The term "PEGylated exenatide variant" is a conjugate of Cys³⁹-exenatide and mPEG-ppMAL(α-methoxy-polyethylene glycol-ω-[3-(3-maleimido-1-oxopropyl)amino] propyl ether), and its structure is as followe: 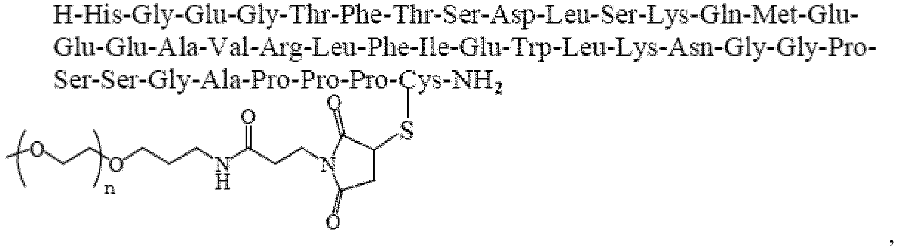 wherein n is independently selected from any natural number, and the molecular weight of the polyethylene glycol modified part falls within the desired range.

The term "molecular weight of polyethylene glycol modified part" refers to the molecular weight of mPEG-ppMAL. Because a polymer is composed of molecules with different polymerization degrees within a certain distribution range, the average molecular weight is generally used to represent the molecular weight of the polymer. Specifically, it may be a number-average molecular weight or may be a weight-average molecular weight. Although there may be some deviation between the number-average molecular weight and the weight-average molecular weight when the polymerization degree of the polymer is quite different, they tend to be equal for a polymer with narrow distribution range. As for the PEGylated exenatide variant of the present invention, when referring to its molecular weight, it might be either the weight-average molecular weight or the number-average molecular weight, and preferably the number-average molecular weight.

In the present invention, the PEGylated exenatide variant comprises a polyethylene glycol modified part and the molecular weight of the polyethylene glycol modified part is not particularly limited, and can be determined by those skilled in the art based on actual needs. In some specific embodiments of the present invention, the molecular weight of the polyethylene glycol modified part is 5-100kDa, preferably 10-50kDa, more preferably 15-40kDa. For example, the molecular weight may be 15kDa, 16kDa, 17kDa, 18kDa, 19kDa, 20kDa, 21kDa, 22kDa, 23kDa, 24kDa, 25kDa, 26kDa, 27kDa, 28kDa, 29kDa, 30kDa, 31kDa, 32kDa, 33kDa, 34kDa, 35kDa, 36kDa, 37kDa, 38kDa, 39kDa, 40kDa and the like.

In some more preferred embodiments of the present invention, the molecular weight of the polyethylene glycol modified part is 20-29kDa.

After the above modification, compared to exenatide, the active ingredients of the present invention have obvious advantages in action duration, bioavailability, therapeutic effect and the like, and have excellent therapeutic effect and safety for diabetic patients.

The pharmaceutical composition of the present invention comprises the above-mentioned active ingredients and pH regulator, and might also be in a dosage form by further comprising other pharmaceutically acceptable carriers, adjuvants or vehicles, etc., so as to facilitate clinical application or storage. The specific dosage form of the pharmaceutical composition is not limited by the present invention, and can be determined by those skilled in the art based on actual needs. For example, the above-mentioned active ingredient, pH regulator and appropriate amount of solvent can be formulated into a liquid preparation, the solvent may be water, ethanol or a mixture of ethanol and water, propylene glycol or a mixture of propylene glycol and water, a mixed solvent of glycerin and water, a mixed solvent of polyethylene glycol and water, peanut oil, sesame oil and soybean oil, etc. More specifically, the liquid preparation may be oral liquid, injection, etc. In some specific embodiments of the present invention, the pharmaceutical composition of the present invention is an injection, and the solvent of the injection can be selected from water (such as water for injection), mixed solvent of ethanol and water, mixed solvent of propylene glycol and water, mixed solvent of polyethylene glycol and water, etc., preferably water for injection.

The content of active ingredient in the pharmaceutical composition is not particularly limited in the present invention, which can be determined by those skilled in the art based on actual needs. In order to ensure excellent therapeutic effect and convenience of administration, in some specific embodiments of the present invention, the concentration of PEGylated exenatide variant in the pharmaceutical composition of the present invention is 0.1-5.0 mg/ml, counting in peptide content.

The inventor of the present invention have found that the concentration of PEGylated exenatide variant also affects the stability of the pharmaceutical composition, and the higher the concentration of the peptide, the better its stability will be. However, when the pharmaceutical composition is an injection, as the concentration of the above peptide increases, the viscosity of the solution also increases, which is not conducive to injection. Therefore, in some preferred embodiments of the present invention, the concentration of PEGylated exenatide variant is not higher than 1.5 mg/ml, which could be specifically 0.2 mg/ml, 0.25 mg/ml, 0.3 mg/ml, 0.35 mg/ml, 0.4 mg/ml, 0.45 mg/ml, 0.5 mg/ml, 0.55 mg/ml, 0.6 mg/ml, 0.65 mg/ml, 0.7 mg/ml, 0.75 mg/ml, 0.8 mg/ml, 0.85 mg/ml, 0.9 mg/ml, 0.95 mg/ml, 1.0 mg/ml, 1.1 mg/ml, 1.2 mg/ml, 1.3 mg/ml, 1.4 mg/ml, 1.5 mg/ml, etc.

When the above concentration is lower than 0.25 mg/ml, the stability of the pharmaceutical composition slightly decreases. Therefore, in some more preferred embodiments of the present invention, the concentration of PEGylated exenatide variant is 0.25-1.5 mg/ml, more preferably 0.3-0.8 mg/ml, and even more preferably 0.4-0.6 mg/ml.

Considering that the stability of the pharmaceutical composition already meet the requirements when the concentration is 0.5 mg/ml, in some preferred embodiments of the present invention, the concentration of PEGylated exenatide variant is 0.5 mg/ml.

### pH

The pH has a significant effect on the stability of PEGylated exenatide variant. The pharmaceutical composition provided by the present invention is more stable under acidic conditions.

In some specific embodiments of the present invention, the pH value of the pharmaceutical composition is 2.0-6.0. Within this pH range, the pharmaceutical composition of the present invention can maintain good stability. When the pH value is above 6.0, PEGylated exenatide variant degrades more rapidly.

In some preferred embodiments of the present invention, the pH value of the pharmaceutical composition is 2.5-5.5, and further preferably is 3.0-5.0. Under these pH condition, the pharmaceutical composition can be stored at low temperatures (such as 4°C) for 9 months, and the purity of the PEGylated exenatide variant detected by liquid chromatography can still be maintained at more than 90%. For example, the pH value may be 3.0, 3.1, 3.2, 3.3, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, etc.

In some more preferred embodiments of the present invention, the pH value of the pharmaceutical composition is 3.5-4.5. If this pH condition could be maintained, the specific pH regulator to be used is not limited, and the pH regulator might be acid or a buffer system, etc. This pH condition can reduce or avoid the negative effect of other component (such as buffer system) on stability.

In some more preferred embodiments of the present invention, when the pH value of the pharmaceutical composition is 3.5-4.5. Under this pH condition, the pharmaceutical composition could be stored at low temperatures (such as 4°C) for 9 months, and the purity of the PEGylated exenatide variant detected by liquid chromatography can still be maintained at more than 94%.

### pH regulator

The type of pH regulator used in the present invention is not particularly limited, and can be determined by those skilled in the art based on actual needs. In some specific embodiments of the present invention, the pH regulator is an acid or a buffer system.

### Acid

When the pH regulator is an acid, the type of the acid is not particularly limited in the present invention, and can be determined by those skilled in the art based on actual needs. For example, it can be citric acid, acetic acid, maleic acid, lactic acid, etc. In some preferred embodiments of the present invention, the acid is selected from citric acid, acetic acid, trifluoroacetic acid, phosphoric acid, hydrochloric acid, tartaric acid, malic acid, propionic acid and a mixture thereof.

In some more preferred embodiments of the present invention, the acid is selected from citric acid, acetic acid, trifluoroacetic acid, phosphoric acid and a mixture thereof.

In some more preferred embodiments of the present invention, the pH regulator is citric acid. This embodiment does not use a buffer system, but only citric acid to adjust the pH value to the above-mentioned desired value (e.g., 3.5-4.5), which can maintain long-term stability of the pharmaceutical composition at high temperature (e.g., 40 °C). The overall stability using citric acid only is even higher than that of the buffer system (such as lactic acid buffer system) used hereinafter.

### Buffer system

Buffer system is composed of a conjugate acid-base pair or an amphoteric compound, which can offset and reduce the effect of additional acid or base on the pH of the solution to a certain extent, thereby maintaining a relatively stable pH of the solution.

When the pH regulator is a buffer system, the type of buffer system used is not particularly limited in the present invention, and can be determined by those skilled in the art based on actual needs. For example, it could be lactic acid/sodium carbonate, lactic acid/sodium hydroxide, potassium dihydrogen phosphate/sodium hydroxide, etc.

In some preferred embodiments of the present invention, the buffer system used in the pharmaceutical composition is selected from the group consisting of glycine/hydrochloric acid, citric acid/sodium citrate, citric acid/sodium dihydrogen phosphate, acetic acid/sodium acetate, lactic acid/sodium hydroxide, lactic acid/sodium carbonate, propionic acid/sodium propionate, succinic acid/sodium succinate and malic acid/sodium hydroxide.

In some more preferred embodiments of the present invention, the buffer system used in the pharmaceutical composition is selected from glycine/hydrochloric acid, acetic acid/sodium acetate, lactic acid/sodium hydroxide, lactic acid/sodium carbonate, propionic acid/sodium propionate, succinic acid/sodium succinate and malic acid/sodium hydroxide. In an appropriate pH range (e.g., pH value is 3.5-4.5), the degradation of PEGylated exenatide variant can be reduced to different extents.

In some more preferred embodiments of the present invention, the buffer system used in the pharmaceutical composition is a lactic acid buffer system, specifically, lactic acid/sodium hydroxide or lactic acid/sodium carbonate. In an appropriate pH range (e.g., pH value is 3.5-4.5), the use of this type of buffer system can maintain good stability of the pharmaceutical composition.

The amount of the above buffer system is not particularly limited in the present invention, and can be determined by those skilled in the art based on actual needs. The amount of the buffer system in the present invention is measured in "concentration of the buffer salt", and the term "concentration of the buffer salt" refers to the concentration of the anion in the buffer system. In some embodiments of the present invention, the concentration of the buffer salt is 10-50 mmol/L, preferably 20-40 mmol/L, more preferably 25-35 mmol/L, and even more preferably 30 mmol/L. The pH is adjusted by changing the proportion of conjugate acid-base pair or amphoteric compound in the buffer system.

The pharmaceutical composition of the present invention can optionally comprise other components, such as osmotic pressure regulator, bacteriostat, etc.

### Osmotic pressure regulator

Whether the osmotic pressure regulator is used or not is not limited in the present invention. In some preferred embodiments of the present invention, the above-mentioned pharmaceutical composition comprises an osmotic pressure regulator. The osmotic pressure regulator is used to regulate the osmotic pressure of the pharmaceutical composition. The type of the osmotic pressure regulator is not particularly limited in the present invention, and it can be a substance commonly used in the art in this field, such as trehalose, mannitol, galactose, maltose, glucose and the like.

In some specific embodiments of the present invention, the osmotic pressure regulator is selected from the group consisting of mannitol, sorbitol, xylitol, glycerol, sodium chloride and a mixture thereof. These osmotic pressure regulators can not only adjust osmotic pressure, but also play an auxiliary role on maintaining the stability of pharmaceutical composition.

In some preferred embodiments of the present invention, the osmotic pressure regulator is mannitol. Compared with the substances mentioned above, such as sodium chloride, mannitol can enhance the stability of the pharmaceutical composition.

The amount of the osmotic pressure regulator is not particularly limited in the present invention, and can be determined by those skilled in the art based on actual needs. In some specific embodiments of the present invention, the concentration of the osmotic pressure regulator is 5-100 mg/ml, preferably 10-80 mg/ml, and more preferably 30-60 mg/ml. For example, the concentration can be specifically 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, 50 mg/ml, 51 mg/ml, 52 mg/ml, 53 mg/ml, 54 mg/ml, 55 mg/ml, 56 mg/ml, 57 mg/ml, 58 mg/ml, 59 mg/ml, 60 mg/ml, etc.

In some preferred embodiments of the present invention, the concentration of the osmotic pressure regulator is 40-50 mg/ml.

### B acterio stat

Whether a bacteriosta is used or not is not limited in the present invention, and can be determined by those skilled in the art based on actual needs. In some specific embodiments of the present invention, the pharmaceutical composition comprises a bacteriosta.

The type of the bacteriosta is not particularly limited in the present invention, and can be determined by those skilled in the art based on actual needs, such as, m-Cresol, phenol, sorbic acid, etc. In some preferred embodiments of the present invention, the bacteriostat is selected from the group consisting of m-Cresol, phenol, chlorocresol, chlorobutanol, benzyl alcohol, paraben and a mixture thereof.

In some more preferred embodiments of the present invention, the bacteriostat is selected from the group consisting of m-Cresol, phenol, chlorocresol, chlorobutanol, paraben and a mixture thereof. By using such bacteriostat, the pharmaceutical composition is more stable.

In some more preferred embodiments of the present invention, when the bacteriostat is m-Cresol, the stability of the pharmaceutical composition is even better.

The amount of the bacteriostat is not particularly limited in the present invention, and can be determined by those skilled in the art based on actual needs. In some specific embodiments of the present invention, the concentration of the bacteriostat is 0.1-5 mg/ml, preferably 1.0-4.0 mg/ml, and more preferably 1.5-3.0 mg/ml. For example, the concentration may be specifically 1.5 mg/ml, 1.6 mg/ml, 1.7 mg/ml, 1.8 mg/ml, 1.9 mg/ml, 2.0 mg/ml, 2.1 mg/ml, 2.2 mg/ml, 2.3 mg/ml, 2.4 mg/ml, 2.5 mg/ml, 2.6 mg/ml, 2.7 mg/ml, 2.8 mg/ml, 2.9 mg/ml, 3.0 mg/ml, etc.

In some preferred embodiments of the present invention, the concentration of the bacteriostat is 2.0-2.5 mg/ml.

### Second aspect of the present invention

In the second aspect, the present invention also provides the use of the above-mentioned pharmaceutical composition.

### Pharmaceutical use

Based on the efficacy of the active ingredient, which is PEGylated exenatide variant capable of controlling blood glucose level, any one of the above-mentioned pharmaceutical composition can effectively and smoothly control blood glucose level. Therefore, the present invention provides the above-mentioned pharmaceutical composition for preventing and/or treating diabetes.

The present invention further provides the use of the above-mentioned pharmaceutical composition in the manufacture of a medicament for preventing and/or treating diabetes.

### Treatment methods

The present invention further provides a method for preventing and/or treating diabetes, which comprises the following steps: administering a prophylactically and/or therapeutically effective amount of the above pharmaceutical composition to a patient in need thereof.

The amount of the pharmaceutical composition administered to the patient will vary depending on the administered drug, the purpose of administration (such as, for preventing or treating), the patient's condition, the route of administration, and other factors. In a therapeutic application, the pharmaceutical composition may be administered to a patient who already has the disease in an amount sufficient to cure or at least partially suppress the symptoms and complications of the disease. The therapeutically effective amount depends on the symptoms of the disease being treated and the following factors judged by the attending clinician, such as the severity of the disease, the patient's age, weight, general condition and the like.

The embodiments of the present invention will be described in detail below in conjunction with examples. However, it will be understood by those skilled in the art that the following examples are only used to illustrate the present invention and should not be regarded as limiting the scope of the present invention. Those examples without indicated specific conditions were carried out according to the conventional conditions or the conditions suggested by the manufacturer. The reagents or instruments used without indicated manufacturer are all conventional products that could be commercially available.

### Example 1: Preparation of PEGylated exenatide variant

### (I) Preparation of the exenatide variant

The synthesis of sulfydryl-containing exenatide variant was carried out by using solid phase carrier of Fmoc-Rink Amide MBHA resins and selecting the Fmoc amino protection strategy.

Step 1: 39-position Cys protected by protecting group and condensing agent HBTU/DIPEA were dissolved in N,N- dimethylformamide (DMF), and the reaction was carried out for 1-5 hours. The completion of the condensation reaction was monitored by ninhydrin method.

Step 2: 10-30% piperidine DMF solution, as the deprotecting agent, was added into the reaction system of the step 1. The reaction was carried out for 10-30 minutes, and the completion of the removal of the amino protecting group was monitored by the ninhydrin method.

Step 3: according to the polypeptide sequence of SEQ ID NO.1, the corresponding amino acids were sequentially used to repeat the Step 1 and Step 2 until all amino acids in the sequence were conjugated to N-terminal.

Step 4: trifluoroacetic acid (TFA) was added into the reaction system as a cleavage agent, the reaction was carried out for 1-5 hours, so that the polypeptide was cleaved from the solid carrier and various protecting groups were removed at the same time.

Step 5: the cleaved peptides in the solution were precipitated with ether; the precipitate was filtered and collected, then passed through C18-type chromatographic column using a mobile phase of 0.1% TFA/acetonitrile-water system for elution. Fraction solutions were collected and then freeze-dried to obtain the exenatide variant.

Step 6: the purity of the product was determined by high-performance liquid chromatography. The molecular weight of the exenatide variant determined by LC-MS was 4201.6, and the theoretical value was 4202.7±2.0.

### (II) Preparation of the PEGylated exenatide variant

Step (1): phosphate buffer solution with pH=6.5 was prepared, filled with nitrogen for 30-50min, and then the exenatide variant and mPEG-ppMAL (the molecular weight of mPEG-ppMAL was about 23kDa, and the feeding molar ratio of exenatide variant to mPEG-ppMAL was 1:2) were added into a reactor filled with the phosphate buffer solution. The reaction temperature was controlled at 22±2°C, the reaction was carried out under stirring for 60 min, and the sample was taken for detection. After the reaction, the reaction solution was diluted with NaAc-HAc solution with pH=4.0, and then the pH value of the mixed solution was adjusted to 4.0 by dropping appropriate glacial acetic acid to obtain a crude solution of PEGylated exenatide variant.

Step (2): the obtained crude solution was loaded on SP ion exchange chromatography column (GE company, XK16/20 column, macroCap SP packing), and subsequently 2 column volumes of 50 mM sodium acetate buffer was added for equilibration. The linear gradient elution was performed with 20 column volumes of 50 mM sodium acetate buffer containing 1M NaCl, and the eluent was collected and freeze-dried to obtain the PEGylated exenatide variant. The number-average molecular weight of the product detected by Malidi-TOF-MS was 27,100.

### Example 2: Screening of pH

60%-70% of the total preparation amount of injection water (≤25°C) was added into a reactor. The desired amount of m-Cresol was weighed and added into the reactor, and the container was washed several times using a proper amount of injection water and all of the liquid was added into the reactor. The mixture was stirred until clear and oil particulate-free. Under slow stirring, the weighed raw material of PEGylated exenatide variant was continuously and slowly added into the reactor, a proper amount of injection water was used to wash the container and added into the reactor to control the amount of liquid preparation to 80%-90% of the total preparation amount, and then the mixture was stirred continuously until the raw material was completely dissolved. Citric acid/sodium citrate solution was used to adjust the pH of the solution to the desired pH, and then the final volume was adjusted. The compositions of the formulations are shown in the following table.

**Table 1. Composition of each formulation in pH screening experiments**

| pH value | Peptide content | m-Cresol | pH regulator | Concentration of buffer salt |
|---|---|---|---|---|
| 3.5 | 0.5mg/ml | 2.2 mg/ml | citric acid/ sodium citrate | 30 mmol/L |
| 4.0 | | | | |
| 4.5 | | | | |
| 5.0 | | | | |
| 5.5 | | | | |
| 6.0 | | | | |
| 6.5 | | | | |

The obtained preparations were placed at 4°C for stability investigation, and samples were taken at the indicated time points to detect the purity (%) of the samples. The purity results are shown in the following table.

**Table 2. Effect of different pH conditions on stability**

| Storage Time | pH value | | | | | | |
|---|---|---|---|---|---|---|---|
| | 3.5 | 4.0 | 4.5 | 5.0 | 5.5 | 6.0 | 6.5 |
| 0d | 98.8 | 98.8 | 98.8 | 98.8 | 98.8 | 98.8 | 98.8 |
| 15d | 98.3 | 98.4 | 98.2 | 98.3 | 98.8 | 98.7 | 97.9 |
| 31d | 97.2 | 97.7 | 97.4 | 97.3 | 98.0 | 97.5 | 95.7 |
| 2M | 97.7 | 98.0 | 97.7 | 97.6 | 98.4 | 96.7 | 94.4 |
| 3M | 97.4 | 97.6 | 97.3 | 96.7 | 97.3 | 95.5 | 92.3 |
| 6M | 97.0 | 97.1 | 96.0 | 95.2 | 95.4 | 92.5 | 84.1 |
| 9M | 96.2 | 96.5 | 94.0 | 93.0 | 93.7 | 89.4 | 78.9 |

Based on the results under different pH conditions in Table 1 and Table 2, it can be seen that the PEGylated exenatide variant was relatively stable under acidic conditions. Among them, the sample was most stable when the pH value was 3.5-4.5, and the sample degraded very quickly when the pH value was above 6.0. Then, confirmatory experiments were carried out at different pH using different buffer systems. The formulations are shown in the following table.

**Table 3. Composition of each formulation in confirmatory experiments of pH screening**

| pH value | Peptide content | m-Cresol | pH regulator | Concentration of buffer salt |
|---|---|---|---|---|
| 3.5 | 0.5 mg/ml | 2.2 mg/ml | citric acid/sodium citrate | 30 mmol/L |
| 4.0 | | | | |
| 4.5 | | | | |
| 5.0 | | | | |
| 4.0 | | | acetic acid/sodium acetate | |
| 4.5 | | | | |
| 5.0 | | | | |
| 3.5 | | | lactic acid/sodium hydroxide | |
| 4.0 | | | | |
| 4.5 | | | | |
| 5.0 | | | | |

The preparation method was as mentioned above. The obtained preparations were divided into three parts and placed at 40°C, 25°C and 4°C respectively for stability investigation. Samples were taken at indicated time points and the purity (%) was detected. The results are shown in Table 4 and Table 5.

**Table 4. Effect of different temperatures on stability**

| **Storage condition** | **Storage time** | **citric acid/sodium citrate (pH)** | | | | **acetic acid/sodium acetate (pH)** | | |
|---|---|---|---|---|---|---|---|---|
| | | **5.0** | **4.5** | **4.0** | **3.5** | **5.0** | **4.5** | **4.0** |
| **40°C** | **0D** | 99.2 | 99.2 | 99.2 | 99.2 | 99.2 | 99.2 | 99.2 |
| | **6D** | 96.5 | 97.2 | 97.9 | 97.5 | 96.5 | 97.5 | 98.0 |
| | **12D** | 93.5 | 95.6 | 96.8 | 97.5 | 93.7 | 95.4 | 96.4 |
| | **23D** | 88.4 | 92.3 | 94.9 | 95.5 | 90.0 | 92.6 | 93.8 |
| | **34D** | 81.6 | 86.7 | 89.7 | 90.2 | 85.3 | 87.2 | 87.8 |
| | **44D** | 64.9 | 71.1 | 74.3 | 75.3 | 78.8 | 79.7 | 80.5 |
| **25°C** | **0D** | 99.2 | 99.2 | 99.2 | 99.2 | 99.2 | 99.2 | 99.2 |
| | **12D** | 97.0 | 97.2 | 97.9 | 98.1 | 96.9 | 97.6 | 98.1 |
| | **35D** | 94.2 | 94.9 | 96.3 | 96.9 | 93.9 | 95.1 | 96.5 |
| | **58D** | 91.7 | 92.0 | 94.3 | 96.0 | 92.5 | 92.4 | 94.8 |
| | **78D** | 88.8 | 90.4 | 93.0 | 94.8 | 90.0 | 90.8 | 93.4 |
| | **111D** | 84.4 | 84.3 | 88.0 | 88.6 | 86.3 | 85.4 | 88.8 |
| | **135D** | 80.3 | 81.3 | 85.6 | 86.7 | 84.3 | 82.7 | 87.0 |
| **4°C** | **0D** | 99.2 | 99.2 | 99.2 | 99.2 | 99.2 | 99.2 | 99.2 |
| | **1M** | 98.4 | 98.2 | 98.9 | 99.0 | 98.5 | 98.7 | 99.1 |
| | **2M** | 98.3 | 98.1 | 98.4 | 98.8 | 98.5 | 98.8 | 99.0 |
| | **3M** | 97.9 | 97.9 | 98.5 | 98.5 | 98.2 | 98.6 | 98.8 |
| | **6M** | 96.9 | 96.9 | 97.7 | 98.2 | 98.5 | 98.2 | 98.5 |

**Table 5. Effect of different temperatures on stability**

| **Storage condition** | **Storage time** | **lactic acid/sodium hydroxide (pH)** | | | |
|---|---|---|---|---|---|
| | | **5.0** | **4.5** | **4.0** | **3.5** |
| **40°C** | **0D** | 99.1 | 99.1 | 99.1 | 99.1 |
| | **6D** | 97.4 | 97.8 | 97.8 | 98.0 |
| | **12D** | 94.5 | 95.9 | 96.1 | 97.0 |
| | **19D** | 92.6 | 94.2 | 93.8 | 94.0 |
| | **25D** | 86.5 | 88.1 | 89.3 | 90.2 |
| | **39D** | 82.9 | 85.6 | 86.2 | 87.6 |
| | **54D** | 76.5 | 78.2 | 80.8 | 83.4 |
| **25°C** | **0D** | 99.1 | 99.1 | 99.1 | 99.1 |
| | **19D** | 96.5 | 97.6 | 97.4 | 97.3 |
| | **39D** | 93.9 | 94.5 | 95.1 | 95.2 |
| | **56D** | 91.3 | 92.7 | 93.5 | 93.8 |
| | **82D** | 88.5 | 90.8 | 90.9 | 89.9 |
| | **104D** | 85.4 | 87.7 | 88.8 | 89.3 |
| | **132D** | 82.8 | 85.4 | 86.8 | 87.4 |
| | **158D** | 79.7 | 83.7 | 84.4 | 85.5 |
| **4°C** | **0D** | 99.1 | 99.1 | 99.1 | 99.1 |
| | **1M** | 98.7 | 98.9 | 99.1 | 99.0 |
| | **2M** | 98.1 | 98.6 | 98.8 | 99.0 |
| | **3M** | 98.1 | 98.5 | 98.6 | 98.9 |
| | **6M** | 96.7 | 97.6 | 97.4 | 97.7 |

Based on the tested results in Table 4 and Table 5, it can be seen that the stability of samples in different buffer systems showed a relatively consistent trend, and all samples were relatively stable at pH=3.5-4.5. Also, it was found that there were certain differences in the sample stability in different buffer systems at the same pH condition. The present invention further screened for the pH regulator.

### Example 3: Screening of pH regulator

60%-70% of the total preparation amount of injection water (≤25°C) was added into the reactor. The desired amount of m-Cresol was weighed and added into the reactor, and the container was washed several times using a proper amount of injection water and all of the liquid was added into the reactor. The mixture was stirred until clear and oil particulate-free. The mannitol was weighed and added into the reactor, and the container was washed several times using a proper amount of injection water and all of the liquid was added into the reactor. The mixture was stirred until mannitol was completely dissolved (if mannitol is not used, this step is omitted). Under slowly stirring, the weighed raw material of PEGylated exenatide variant was continuously and slowly added into the reactor, a proper amount of injection water was used to wash the container and added into the reactor to control the amount of liquid preparation to 80%-90% of the total preparation amount. The mixture was stirred continuously until the raw material was completely dissolved. The pH regulator was used to adjust the pH value of the solution to the desired value, and then the final volume was adjusted.

### (1) pH=4.5

The preparation process was performed twice according to the above method by using same batch of raw materials, and the formulations are shown in the following table.

**Table 6. Composition of each formulation in pH regulator screening experiments**

| Batch | pH regulator | Peptide content | Mannitol | m-Cresol | Concentration of buffer salt | pH value |
|---|---|---|---|---|---|---|
| first preparation | acetic acid/sodium acetate | 0.5mg/ml | 41mg/ml | 2.2mg/ml | 30mmol/L | 4.50 |
| | succinic acid/sodium succinate | | | | | |
| | propionic acid/sodium propionate | | | | | |
| | citric acid/sodium citrate | | | | | |
| | lactic acid/sodium carbonate | | | | | |
| | malic acid/sodium hydroxide | | | | | |
| second preparation | acetic acid/sodium acetate | 0.5mg/ml | None | 2.2mg/ml | 30mmol/L | 4.50 |
| | lactic acid/sodium carbonate | | | | | |
| | lactic acid/sodium hydroxide | | | | | |

The obtained preparations were divided into three parts and placed at 40°C, 25°C and 4°C respectively for stability investigation. Samples were taken at indicated time points for purity (%) detection. The results are shown in Table 7.

**Table 7. Effect of different pH regulators on stability**

| **Stora ge condi tion** | **storag e time** | First preparation | | | | | | Second preparation | | | |
|---|---|---|---|---|---|---|---|---|---|---|---|
| | | acetic acid/so dium acetate | succini c acid/so dium succin ate | propio nic acid/so dium propio nate | citric acid/so dium citrate | lactic acid/so dium carbon ate | malic acid /sodiu m hydrox ide | **storag e time** | acetic acid/so dium acetate | lactic acid/so dium carbon ate | lactic acid/sodi um hydroxid e |
| **40°C** | **0D** | 98.9 | 98.9 | 98.9 | 98.9 | 98.9 | 98.9 | **0D** | 99.3 | 99.3 | 99.3 |
| | **6D** | 96.6 | 97.0 | 96.9 | 97.1 | 98.2 | 97.9 | **7D** | 96.8 | 97.5 | 97.5 |
| | **12D** | 94.4 | 95.3 | 95.6 | 94.8 | 96.6 | 96.0 | **19D** | 91.7 | 94.2 | 94.4 |
| | **20D** | 91.8 | 93.1 | 93.3 | 92.3 | 94.9 | 93.9 | **34D** | 84.8 | 90.3 | 89.9 |
| | **38D** | 86.4 | 88.5 | 88.9 | 85.7 | 90.8 | 88.6 | **50D** | 76.5 | 82.2 | 81.3 |
| | **52D** | 83.0 | 86.0 | 84.6 | 69.3 | 87.6 | 85.0 | **64D** | 72.7 | 78.5 | 77.2 |
| | **67D** | 79.2 | 82.5 | 81.0 | 63.5 | 84.9 | 81.7 | **85D** | 65.5 | 71.3 | 69.3 |
| **25°C** | **0D** | 98.9 | 98.9 | 98.9 | 98.9 | 98.9 | 98.9 | **0D** | 99.3 | 99.3 | 99.3 |
| | **14D** | 97.3 | 97.4 | 97.8 | 97.3 | 98.0 | 98.0 | **25D** | 96.5 | 97.6 | 97.6 |
| | **38D** | 94.1 | 95.1 | 95.8 | 94.2 | 96.3 | 95.9 | **57D** | 92.8 | 94.1 | 94.3 |
| | **52D** | 93.1 | 94.4 | 94.8 | 92.6 | 95.4 | 94.9 | **89D** | 88.3 | 94.3 | 92.3 |
| | **67D** | 92.3 | 93.0 | 94.3 | 90.8 | 94.8 | 93.7 | **116D** | 85.2 | 89.8 | 90.1 |
| | **87D** | 90.0 | 90.2 | 92.0 | 86.7 | 92.0 | 90.7 | **138D** | 83.2 | 88.7 | 88.6 |
| | **108D** | 87.9 | 88.0 | 90.7 | 84.0 | 90.7 | 88.6 | **183D** | 75.9 | 82.2 | 82.6 |
| | **137D** | 85.2 | 85.1 | 88.6 | 70.9 | 88.5 | 86.1 | | | | |
| **4°C** | **0D** | 98.9 | 98.9 | 98.9 | 98.9 | 98.9 | 98.9 | **0D** | 99.3 | 99.3 | 99.3 |
| | **12D** | 98.4 | 98.5 | 98.5 | 98.3 | 99.0 | 98.0 | **1M** | 99.0 | 99.0 | 98.9 |
| | **38D** | 99.2 | 99.5 | 98.3 | 98.2 | 98.8 | 98.6 | **2M** | 98.5 | 99.0 | 98.9 |
| | **2M** | 98.2 | 98.3 | 98.3 | 97.9 | 98.6 | 98.6 | **3M** | 98.5 | 98.7 | 98.54 |
| | **3M** | 98.1 | 98.3 | 98.2 | 97.8 | 98.7 | 98.5 | **6M** | 98.1 | 98.4 | 98.2 |
| | **6M** | 98.3 | 98.0 | 98.0 | 97.2 | 98.7 | 98.3 | | | | |
| | **9M** | 97.9 | 97.7 | 98.1 | 96.5 | 98.2 | 97.8 | | | | |

According to the results shown in Table 7, the degradation rate of the active ingredient was faster in the citrate buffer system. In the lactate buffer system, the stability of the active ingredient was relatively good. The other buffer systems had a similar effect on the stability of the active ingredient.

Compared with the first preparation, the osmotic pressure regulator was omitted in the second preparation. According to the results shown in Table 7, in the same buffer system, when the osmotic pressure regulator is omitted, the degradation rate of the active ingredient was increased. This demonstrated that the osmotic pressure regulator not only had the function of regulating osmotic pressure, but also played an auxiliary role on maintaining the stability of the preparation.

### (2) pH=4.0

The preparation was performed according to the above method by using three batches of raw materials, and the composition of each formulation is shown in the following table.

**Table 8. Composition of each formulation in pH regulator screening experiments**

| Batch | pH regulator | Peptide content | m-Cresol | Concentration of buffer salt | pH |
|---|---|---|---|---|---|
| 1 | acetic acid/sodium acetate | 0.5mg/ml | 2.2mg/ml | 30mmol/L | 4.0 |
| | succinic acid/sodium succinate | | | | |
| | citric acid/sodium citrate | | | | |
| | lactic acid/sodium hydroxide | | | | |
| | lactic acid/sodium carbonate | | | | |
| 2 | citric acid | 0.5mg/ml | 2.2mg/ml | / | 4.0 |
| | citric acid/sodium citrate | | | 30mmol/L | |
| | acetic acid/sodium acetate | | | | |
| | lactic acid/sodium hydroxide | | | | |
| | succinic acid/sodium succinate | | | | |
| 3 | citric acid | 0.5mg/ml | 2.2mg/ml | / | 4.0 |
| | acetic acid/sodium acetate | | | 30mmol/L | |
| | lactic acid/sodium hydroxide | | | | |

The obtained preparations were placed at 40°C for stability investigation. Samples were taken at the indicated time points and the purity (%) was detected. The results are shown in Table 9.

**Table 9. Effect of different pH regulators on stability**

| **Batch** | **Storage time** | **pH regulator** | | | | | |
|---|---|---|---|---|---|---|---|
| | | **acetic acid/sodi um acetate** | **succinic acid/sodi um succinate** | **citric acid/sodi um citrate** | **lactic acid/so dium carbon ate** | **lactic acid/sodi um hydroxid e** | **citric acid** |
| **1** | **0D** | 98.9 | 98.9 | 98.9 | 98.9 | 98.9 | / |
| | **7D** | 97.9 | 97.9 | 98.1 | 98.0 | 98.3 | / |
| | **22D** | 94.0 | 93.7 | 95.5 | 95.4 | 94.9 | / |
| | **36D** | 88.1 | 88.1 | 90.9 | 91.5 | 90.4 | / |
| | **47D** | 84.5 | 84.5 | 87.0 | 87.6 | 86.6 | / |
| | **61D** | 78.5 | 78.6 | 68.5 | 82.9 | 82.4 | / |
| **2** | **0D** | 98.2 | 98.2 | 98.2 | / | 98.2 | 98.2 |
| | **7D** | 96.8 | 96.8 | 97.4 | / | 96.9 | 96.1 |
| | **14D** | 94.0 | 95.1 | 96.3 | / | 95.7 | 95.2 |
| | **21D** | 90.1 | 91.3 | 94.0 | / | 91.2 | 91.6 |
| | **28D** | 91.5 | 92.9 | 94.8 | / | 93.2 | 94.1 |
| | **35D** | 87.0 | 88.4 | 81.6 | / | 88.7 | 89.2 |
| | **47D** | 82.7 | 84.9 | 77.9 | / | 84.1 | 85.2 |
| | **58D** | 74.5 | 81.4 | 72.7 | / | 82.3 | 81.5 |
| **3** | **0D** | 97.9 | / | / | / | 97.9 | 97.9 |
| | **7D** | 96.4 | / | / | / | 96.4 | 96.5 |
| | **14D** | 95.1 | / | / | / | 94.9 | 95.2 |
| | **21D** | 93.7 | / | / | / | 93.9 | 94.0 |
| | **28D** | 92.5 | / | / | / | 93.0 | 93.0 |
| | **35D** | 91.6 | / | / | / | 90.9 | 91.8 |
| | **48D** | 89.8 | / | / | / | 89.5 | 89.4 |
| | **63D** | 82.5 | / | / | / | 81.7 | 85.6 |
| | **78D** | 79.8 | / | / | / | 79.0 | 81.5 |

From the results shown in Table 9, it can be seen that, when the pH was set at pH=4.0, the effects of various pH regulators on stability showed the same trend as at pH=4.5. Among them, the active ingredient was degraded fast in citrate buffer system, while maintained a good stability in the lactate buffer system. Unexpectedly, when pH was adjusted to the desired value using citric acid only, without adding buffer system, the purity of the active ingredient in the preparation still maintained high after long-term storage at high temperature, and the overall stability is even higher than that using lactic acid buffer system. This demonstrated that under suitable pH conditions, even without adding buffer system, the obtained preparation can achieve the equivalent or even higher stability than that with lactic acid buffer system.

### Example 4: Screening of peptide concentration

60%-70% of the total preparation amount of injection water (≤25°C) was added into the reactor. The desired amount of m-Cresol was weighed and added into the reactor, and the container was washed several times using a proper amount of injection water and all of the liquid was added into the reactor. The mixture was stirred until clear and oil particulate-free. The mannitol was weighed and added into the reactor, and the container was washed several times using a proper amount of injection water and all of the liquid was added into the reactor. The mixture was stirred until mannitol was completely dissolved. Under slowly stirring, the weighed raw material of PEGylated exenatide variant was continuously and slowly added into the reactor, a proper amount of injection water was used to wash the container and added into the reactor to control the amount of liquid preparation to 80%-90% of the total preparation amount. The mixture was stirred continuously until the raw material was completely dissolved. The pH regulator, i.e. citric acid solution, was used to adjust the pH value of the solution to the desired value, and then the final volume was adjusted. The compositions of formulas are shown in the following table.

**Table 10. Compositions of the formulations in peptide concentration screening experiments**

| pH regulator | Peptide concentration | pH | Mannitol | m-Cresol |
|---|---|---|---|---|
| citric acid | 0.25mg/mL | 3.8 | 41mg/mL | 2.2mg/mL |
| | 0.5mg/mL | | | |
| | 1.5mg/mL | | | |

The obtained preparations were placed at 40°C for stability investigation. Samples were taken at indicated time points for purity (%) detection. The results are shown in Table 11.

**Table 11. Effect of different peptide concentrations on stability**

| **40°C** | | | | | | | |
|---|---|---|---|---|---|---|---|
| **Peptide concentration** | **0D** | **7D** | **14D** | **21D** | **30D** | **44D** | **59D** |
| **0.25mg/ml** | 97.8 | 95.6 | 93.4 | 90.0 | 88.8 | 83.4 | 79.6 |
| **0.5mg/ml** | 97.8 | 96.2 | 94.0 | 90.6 | 88.8 | 85.7 | 81.3 |
| **1.5mg/ml** | 97.8 | 96.6 | 94.6 | 92.1 | 90.3 | 86.3 | 83.1 |

From the data shown in Table 11, it can be seen that the higher the peptide concentration in the preparation, the better the stability. However, considering that the viscosity of the solution increases when the peptide concentration is higher than 1.5 mg/mL, which is not conducive to injection. When the peptide concentration is 0.5mg/mL, the stability of the preparation has already met the demand, therefore the preferred peptide concentration of the present invention is 0.5 mg/mL.

### Example 5: Screening of osmotic pressure regulator

60%-70% of the total preparation amount of injection water (≤25°C) was added into the reactor. The desired amount of m-Cresol was weighed and added into the reactor, and the container was washed several times using a proper amount of injection water and all of the liquid was added into the reactor. The mixture was stirred until clear and oil particulate-free. The osmotic pressure regulator was weighed and added into the reactor, and the container was washed several times using a proper amount of injection water and all of the liquid was added into the reactor. The mixture was stirred until the osmotic pressure regulator was completely dissolved. Under slowly stirring, the weighed raw material of PEGylated exenatide variant was continuously and slowly added into the reactor, a proper amount of injection water was used to wash the container and added into the reactor to control the amount of liquid preparation to 80%-90% of the total preparation amount. The mixture was stirred continuously until the raw material was completely dissolved. The pH regulator-citric acid solution was used to adjust the pH value of the solution to the desired value, and then the final volume was adjusted. The compositions of formulations are shown in the following table.

**Table 12. Composition of each formulation in pressure regulator osmotic screening experiments**

| pH regulator | Osmotic pressure regulator | pH value | Peptide concentration | m-Cresol |
|---|---|---|---|---|
| Citric acid | 41mg/ml mannitol | 4.5 | 0.5mg/ml | 2.2mg/ml |
| | 0.9% sodium chloride | | | |

The obtained preparations were placed at 40°C for stability investigation. Samples were taken at indicated time points for purity (%) detection. The results are shown in Table 13.

**Table 13. Effect of different osmotic pressure regulators on stability**

| 40°C | | | | | | | |
|---|---|---|---|---|---|---|---|
| Osmotic pressure regulator | 0D | 7D | 14D | 28D | 35D | 42D | 49D |
| Mannitol | 97.2 | 95.1 | 93.9 | 91.2 | 89.1 | 84.6 | 83.3 |
| Sodium chloride | 97.2 | 92.1 | 88.2 | 81.8 | 77.9 | 74.7 | 72.2 |

From the data shown in Table 13, it can be seen that the stability of the preparation with mannitol is better than that with sodium chloride.

Then, part of mannitol was replaced with glycine and the stability of the preparation was further investigated. The composition of each formulation is shown in the following table.

**Table 14 Composition of each formulation in the comparative investigation of glycine and mannitol**

| Number | pH regula tor | Buffer salt concent ration | Glycine (mg/ml) | Mannitol (mg/ml) | pH value | Peptide concentr ation | m-Cresol |
|---|---|---|---|---|---|---|---|
| 1 | acetic acid / sodiu m acetat e | 30 mmol/L | 17.72 | / | 4.5 | 0.5 mg/ml | 2.2 mg/ml |
| 2 | | | 15.50 | 5.38 | | | |
| 3 | | | 13.29 | 10.75 | | | |
| 4 | | | 8.86 | 21.5 | | | |
| 5 | | | / | 43 | | | |
| 6 | | | / | 41 | | | |
| 7 | | | / | / | | | |

The obtained preparations were placed at 25°C and 40°C respectively for stability investigation. Samples were taken at indicated time points for purity (%) detection. The results are shown in Table 15.

**Table 15. Effect of glycine and mannitol on stability**

| Storage condition | | 1 | 2 | 3 | 4 | 5 | 6 | 7 |
|---|---|---|---|---|---|---|---|---|
| 0D | | 99.34 | 99.37 | 99.36 | 99.30 | 99.31 | 99.29 | 99.30 |
| 25°C | 4d | 98.55 | 98.69 | 98.99 | 99.14 | 99.18 | 99.20 | 98.99 |
| | 10d | 97.24 | 98.07 | 98.50 | 98.85 | 99.03 | 99.03 | 98.68 |
| | 15d | 95.06 | 97.23 | 97.91 | 98.50 | 98.79 | 98.80 | 98.36 |
| | 21d | 92.11 | 94.16 | 96.53 | 98.18 | 98.66 | 98.66 | 98.01 |
| 40°C | 4d | 93.75 | 96.20 | 97.16 | 98.40 | 98.87 | 98.83 | 98.31 |
| | 10d | 76.57 | 83.68 | 91.45 | 93.24 | 97.08 | 96.99 | 96.00 |
| | 15d | 62.91 | 71.57 | 82.46 | 88.45 | 95.96 | 96.78 | 95.29 |
| | 21d | 48.29 | 57.25 | 67.96 | 81.28 | 95.47 | 95.53 | 92.52 |

From the data shown in Table 15, it can be seen that the preparations with only mannitol added (formulas 5 and 6) had the best stability, while the preparation with only glycine added (formula 1) had the worst stability, degrading fastest at both 25°C and 40°C. These results indicated that glycine had a destructive effect on the sample, while mannitol had a certain protective effect on the sample and can enhance its stability. Therefore, mannitol is used as an osmotic pressure regulator in the present invention.

### Example 6: Screening of bacteriostat

60%-70% of the total preparation amount of injection water (≤25°C) was added into the reactor. The desired amount of bacteriostat was weighed and added into the reactor, and the container was washed several times using a proper amount of injection water and all of the liquid was added into the reactor. The mixture was stirred until clear and oil particulate-free. The mannitol was weighed and added into the reactor, and the container was washed several times using a proper amount of injection water and all of the liquid was added into the reactor. The mixture was stirred until the mannitol was completely dissolved. Under slowly stirring, the weighed raw material of PEGylated exenatide variant was continuously and slowly added into the reactor, a proper amount of injection water was used to wash the container and added into the reactor to control the amount of liquid preparation to 80%-90% of the total preparation amount. The mixture was stirred continuously until the raw materials were completely dissolved. The pH regulator was used to adjust the pH value of the solution to the desired value, and then the final volume was adjusted. The composition of each formula is shown in the following table.

**Table 16. Composition of each formulation in the bacteriostat screening experiments**

| pH regulator | Mannitol | pH value | Peptide concentration | B acterio stat |
|---|---|---|---|---|
| acetic acid/sodium acetate | 43 mg/ml | 4.5 | 0.5 mg/ml | 2.2 mg/ml m-Cresol |
| | | | | 2.2 mg/ml benzyl alcohol |

The obtained preparations were placed at 25°C and 40°C respectively for stability investigation. Samples were taken at indicated time points for purity (%) detection. The results are shown in Table 17.

**Table 17. Effect of different bacteriostats on stability**

| Storage condition | | m-Cresol | Benzyl alcohol |
|---|---|---|---|
| 0D | | 99.35 | 97.21 |
| 25°C | 7d | 99.24 | 79.44 |
| | 10d | 99.13 | 75.40 |
| | 15d | 99.01 | 72.76 |
| | 21d | 98.65 | 71.54 |
| 40°C | 7d | 97.11 | 74.18 |
| | 10d | 96.17 | 73.59 |
| | 15d | 94.96 | 70.81 |
| | 21d | 91.69 | 65.47 |

From the data shown in Table 17, it can be seen that the purity of the sample with benzyl alcohol as bacteriostat decreased rapidly, while the sample with m-Cresol as bacteriostat was more stable.

### Example 7: Investigation on stability of the composition comprising PEGylated exenatide variant

60%-70% of the total preparation amount of injection water (≤25°C) was added into the reactor. The desired amount of m-Cresol was weighed and added into the reactor, and the container was washed several times using a proper amount of injection water and all of the liquid was added into the reactor. The mixture was stirred until clear and oil particulate-free. The mannitol was weighed and added into the reactor, and the container was washed several times using a proper amount of injection water and all of the liquid was added into the reactor. The mixture was stirred until the mannitol was completely dissolved. Under slowly stirring, the weighed raw material of PEGylated exenatide variant was continuously and slowly added into the reactor, a proper amount of injection water was used to wash the container and added into the reactor to control the amount of liquid preparation to 80%-90% of the total preparation amount. The mixture was stirred continuously until the raw material was completely dissolved. The citric acid was used to adjust the pH value of the solution to desired value, and then the final volume was adjusted.

Two batches of raw materials were used for preparation according to the formulations shown in Table 18.

**Table 18 The composition of the formulation**

| pH value | Peptide content | Mannitol | pH regulator | m-Cresol |
|---|---|---|---|---|
| 3.5-4.5 | 0.5 mg/ml | 47 mg/ml | citric acid | 2.2 mg/ml |

The obtained preparations were placed at 25°C and 40°C respectively for stability investigation. Samples were taken at indicated time points for purity (%) detection. The results are shown in Table 19.

**Table 19. Results of stability investigation**

| Lot No. | pH measured in 0 M | Storage condition | Purity | |
|---|---|---|---|---|
| | | | 0 M | 6 M |
| 1 | 4.15 | 2-8°C | 99.02 | 98.20 |
| | | 25°C | | 90.40 |
| 2 | 4.01 | 2-8°C | 98.90 | 97.80 |
| | | 25°C | | 90.70 |

From the results shown in Table 19, it can be seen that the purity of the the PEGylated exenatide variant in the formulation provided by the present invention was above 90% after long-term storage at low temperature (2-8°C) and regular temperature (25°C). Especially at low temperature (2-8°C), the degradation of active ingredient was very little. This demonstrates that the preparation provided by the present invention is suitable for long-term storage.

Described above are embodiments of the present invention, which are exemplary rather than exhaustive, and is not limited to what is disclosed in the embodiments. Various modifications and changes are apparent for those of ordinary skill in the art, without departing from the scope and spirit of the described embodiments. The choice of terms used herein is intended to best explain the principles, practical applications, or technical improvements in the market of each embodiment, or to enable other ordinary skilled in the art to understand the embodiments disclosed herein.

## Claims

1. A pharmaceutical composition comprising a PEGylated exenatide variant and a pH regulator,
wherein the structure of the PEGylated exenatide variant is: 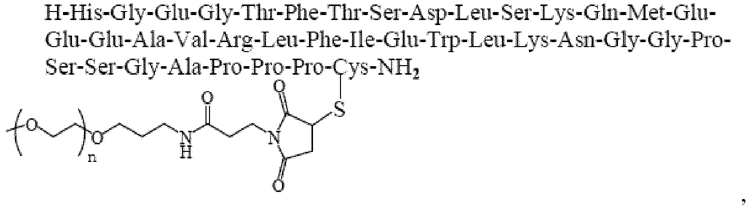 and
the PEGylated exenatide variant comprises a polyethylene glycol modified part and molecular weight of the polyethylene glycol modified part is 5-100kDa, preferably 10-50kDa, more preferably 15-40kDa, and even more preferably 20-29 kDa.

2. The pharmaceutical composition according to claim 1, wherein the pharmaceutical composition comprises a solvent.

3. The pharmaceutical composition according to claim 2, wherein the pH of the pharmaceutical composition is 2.0 to 6.0, preferably 2.5 to 5.5, more preferably 3.0 to 5.0, and even more preferably 3.5 to 4.5.

4. The pharmaceutical composition according to any one of claims 1-3, wherein the pH regulator is an acid;
preferably, the acid is selected from the group consisting of citric acid, acetic acid, trifluoroacetic acid, phosphoric acid, hydrochloric acid, tartaric acid, malic acid, propionic acid and a mixture thereof,
more preferably the acid is selected from the group consisting of citric acid, acetic acid, trifluoroacetic acid, phosphoric acid and a mixture thereof, and
even more preferably the acid is citric acid.

5. The pharmaceutical composition according to any one of claims 1-3, wherein the pH regulator is a buffer system;
preferably, the buffer system is selected from the group consisting of glycine/hydrochloric acid, citric acid/sodium citrate, citric acid/disodium hydrogen phosphate, acetic acid/sodium acetate, lactic acid/sodium hydroxide, lactic acid/sodium carbonate, propionic acid/sodium propionate, succinic acid/sodium succinate and malic acid/sodium hydroxide,
more preferably, the buffer system is selected from the group consisting of glycine/hydrochloric acid, acetic acid/sodium acetate, lactic acid/sodium hydroxide, lactic acid/sodium carbonate, propionic acid/sodium propionate, succinic acid/sodium succinate and malic acid/sodium hydroxide, and
even more preferably, the buffer system is selected from the group consisting of lactic acid/sodium hydroxide and lactic acid/sodium carbonate.

6. The pharmaceutical composition according to any one of claims 2-5, wherein the concentration of the PEGylated exenatide variant is 0.1-5.0 mg/ml counting in peptide content, preferably 0.25-1.5 mg/ml, more preferably 0.3-0.8 mg/ml, even more preferably 0.4-0.6 mg/ml, and most preferably 0.5 mg/ml.

7. The pharmaceutical composition according to any one of claims 1-6, wherein the pharmaceutical composition comprises an osmotic pressure regulator;
preferably, the osmotic pressure regulator is selected from the group consisting of mannitol, sorbitol, xylitol, glycerine, sodium chloride and a mixture thereof, more preferably mannitol; and
preferably, the concentration of the osmotic pressure regulator is 5-100 mg/ml, preferably 10-80 mg/ml, more preferably 30-60 mg/ml, and even more preferably 40-50 mg/ml.

8. The pharmaceutical composition according to any one of claims 1-7, wherein the pharmaceutical composition further comprises a bacteriostat;
preferably, the bacteriostat is selected from the group consisting of m-Cresol, phenol, chlorocresol, chlorobutanol, benzyl alcohol, paraben and a mixture thereof,
more preferably, the bacteriostat is selected from the group consisting of m-Cresol, phenol, chlorocresol, chlorobutanol, paraben and a mixture thereof, and
even more preferably the bacteriostat is m-Cresol; and
preferably, the concentration of the bacteriostat is 0.1-5 mg/ml, preferably 1.0-4.0 mg/ml, more preferably 1.5-3.0 mg/ml, and even more preferably 2.0-2.5 mg/ml.

9. The pharmaceutical composition according to any one of claims 1-8, wherein the pharmaceutical composition is in a dosage form of an injection.

10. Use of the pharmaceutical composition according to any one of claims 1-9 in the manufacture of a medicament for preventing and/or treating diabetes.
